# EUROPÄISCHE PATENTSCHRIFT

(11) **EP 3 176 251 B1**
(45) Veröffentlichungstag und Bekanntmachung des Hinweises auf die Patenterteilung: **20.11.2019**
(21) Anmeldenummer: 16202052.3
(22) Anmeldetag: 02.12.2016
(51) Int. Cl.: C12M 1/107, C12M 1/06

(54) **BEHÄLTER MIT REVISIONSÖFFNUNG**
CONTAINER WITH MAINTENANCE OPENING
RÉCIPIENT DOTÉ D'UNE TRAPPE D'INSPECTION

(30) Priorität: 03.12.2015 DE 102015121108
(43) Veröffentlichungstag der Anmeldung: 07.06.2017
(73) Patentinhaber: Thürwächter GmbH & Co. KG, 87477 Sulzberg (DE)
(72) Erfinder: Thürwächter, Paul, 87477 Sulzberg (DE)
(74) Vertreter: Patentanwälte Olbricht, Buchold, Keulertz Partnerschaft mbB

(56) Entgegenhaltungen:
- EP-A1- 1 130 084
- DE-A1- 3 345 643
- DE-A1-102007 012 014
- DE-A1-102009 034 127
- DE-A1-102012 021 206
- DE-U1-202004 011 561

## Beschreibung

Die Erfindung betrifft einen Behälter, ein Verfahren zum Entnehmen eines Rührwerks und eine Verwendung.

Behälter werden beispielsweise als Fermenter für Biogasanlagen oder zum Lagern bzw. Zersetzen von Gülle eingesetzt. Derartige Behälter sind typischerweise mit einem Rührwerk ausgestattet, welches für eine Durchmischung eines im Behälter befindlichen Biogassubstrats oder der Gülle sorgt. Damit können beispielsweise eine erwünschte Zersetzung durch Bakterien und eine ebenfalls erwünschte Erzeugung von Biogas verbessert werden, da Inhomogenitäten im Substrat oder in der Gülle aufgelöst werden können. Beispielhaft zeigt Dokument DE 20 2004 011 561 U1 einen Behälter mit einem Rührwerk, das jedoch nicht entnehmbar ist. Dokument
US2013/0170315 A offenbart einen Biogasbehälter, der ein Rührwerk aufweist, welches durch eine in der Behälterwand befindlichen Revisionsöffnung herausbefördert werden kann, wie z.B. zu Wartungszwecken.

Problematisch an der Verwendung derartiger Rührwerke ist, dass diese regelmäßig zur Wartung oder auch bei Fehlfunktionen zu entnehmen sind. Da die hier relevanten Behälter typischerweise oben mit einem Deckel oder mit einer Abdeckung verschlossen sind, um Biogas einzufangen und übermäßige Geruchsbelästigung zu vermeiden, ist dies bei Behältern gemäß dem Stand der Technik mit einem erheblichen Aufwand verbunden. Typischerweise sind ein aufwändiges Abnehmen des schweren Deckels, ein Verbringen eines Krans über den Behälter, ein Befestigen des Rührwerks am Kran und ein Herausheben des Rührwerks mittels des Krans oben aus dem Behälter erforderlich.

Um die Entnahme eines Rührwerks zu erleichtern wurde bereits vorgeschlagen, in einer Behälterwand eine Revisionsöffnung vorzusehen, welche zum Entnehmen des Rührwerks geöffnet werden kann. Der Aufwand zum Entnehmen des Rührwerks ist jedoch auch bei einer solchen Ausführung sehr hoch, da hierzu ein externer Kran nötig ist.

Es ist deshalb eine Aufgabe der Erfindung, einen Behälter vorzusehen, bei welchem das Rührwerk leichter entnommen werden kann. Es sind des Weiteren Aufgaben der Erfindung, ein zugehöriges Verfahren sowie eine zugehörige Verwendung vorzusehen.

Dies wird erfindungsgemäß durch einen Behälter, ein Verfahren sowie eine Verwendung gemäß den jeweiligen Hauptansprüchen erreicht. Vorteilhafte Ausgestaltungen können beispielsweise den jeweiligen Unteransprüchen entnommen werden.

Die Erfindung betrifft einen Behälter, insbesondere einen Fermenter für eine Biogasanlage, der eine Behälterwand, welche den Behälter seitlich umschließt, aufweist. Der Behälter weist ein Rührwerk auf, welches einen Motor und einen damit verbundenen Propeller aufweist. Mittels dieses Rührwerks ist insbesondere eine vorteilhafte Durchmischung von in dem Behälter befindlichem Biomassesubstrat oder Gülle möglich. Der Behälter weist eine Rührwerkshalterung auf, welche dazu ausgebildet ist, das Rührwerk zu tragen und an unterschiedliche Positionen innerhalb des Behälters zu verbringen.

Es ist vorgesehen, dass in der Behälterwand eine verschließbare Revisionsöffnung ausgebildet ist, durch welche das Rührwerk entnehmbar ist. Die Rührwerkshalterung ist dazu ausgebildet, das Rührwerk in eine Position horizontal neben der Revisionsöffnung zu verbringen.

Mittels des wie bislang beschrieben ausgeführten Behälters kann auf das gemäß dem Stand der Technik häufig erforderliche aufwändige Abmontieren eines Behälterdeckels sowie auf das Verbringen eines Krans über den Behälter verzichtet werden. Vielmehr kann hierzu die seitliche Revisionsöffnung verwendet werden, was den Aufwand deutlich verringert.

Erfindungsgemäß ist vorgesehen, dass außenseitig oder innenseitig an der Behälterwand, benachbart zur Revisionsöffnung eine Galgenhalterung für einen Galgen angebracht ist, wobei in der Galgenhalterung ein Galgen befestigt ist, welcher einen zumindest teilweise über der Revisionsöffnung liegenden Querbalken aufweist, und wobei der Galgen um eine vertikale Achse schwenkbar ist

Die erfiundungsgemäße Ausführung erleichtert das Entnehmen des Rührwerks, da kein externer Kran oder andere externe Hebevorrichtung benötigt wird. Der Behälter hat vielmehr bereits einen daran befestigten Galgen, um für allfällige Wartungsarbeiten das Rührwerk einfacch und kraftsparend zu entnehmen. Der Bedarf an extra für solche Wartungsarbeiten heranzuführenden Komponenten - insbesondere an schweren Komponenten wie einem Kran - wird auf diese Weise minimiert.

Der Galgen ist um eine vertikale Achse schwenkbar. Damit kann das Rührwerk aus dem Behälter herausgeschwenkt werden. Dies ermöglicht eine besonders einfache Entnahme des Rührwerks durch eine einfache Schwenkbewegung.

Bevorzugt ist die Rührwerkshalterung dazu ausgebildet, dass das Rührwerk benachbart zur Revisionsöffnung von der Rührwerkshalterung entfernbar ist. Damit wird ein Entnehmen des Rührwerks erleichtert. Beispielsweise kann ein Verschluss vorgesehen sein, welcher einfach geöffnet werden kann.

Die Rührwerkshalterung kann insbesondere als vertikaler Mast ausgebildet sein, entlang welchem das Rührwerk verfahrbar ist. Ein solcher Mast kann beispielsweise auch schräg ausgebildet sein.

Bevorzugt ist die Rührwerkshalterung zum Schwenken des Rührwerks um eine vertikale Achse oder um eine Achse des Masts ausgebildet. Damit kann in vorteilhafer Weise eingestellt werden, in welcher Richtung die Rührwirkung erreicht werden soll, beipsielsweise um verfestigte Bereiche gezielt aufzuösen.

Gemäß einer bevorzugten Ausführung trägt der Galgen eine Hebevorrichtung, insbesondere eine Horizontaltraverse, welche zum Befestigen des im Behälter befindlichen Rührwerks durch die geöffnete Revisionsöffnung teilweise in den Behälter hineinreicht. Dies erleichtert das Befestigen des Rührwerks. Es sei verstanden, dass ein solcher Zustand insbesondere während eines Entnehmens oder Wiedereinsetzens des Rührwerks auftritt.

Die Hebevorrichtung kann vorteilhaft um eine horizontale Achse schwenkbar sein. Damit kann sie beispielsweise auf die nachfolgend beschriebene Art und Weise ins Gleichgewicht gebracht werden. Des Weiteren ist insbesondere auch eine Verschwenkbarkeit in einer vorzugsweise horizontal orientierten Ebene vorgesehen.

Gemäß einer Weiterbildung ist die Horizontaltraverse an einem ihrer Enden mit dem Rührwerk verbindbar und an einem gegenüberliegenden Ende mit einem Gegengewicht verbindbar, so dass die Horizontaltraverse zumindest im Wesentlichen ausbalanciert ist. Dies erleichtert die Handhabung.

Das Gegengewicht kann vorzugsweise ein Behälter sein, welcher mit Ballaststoff, insbesondere mit Wasser, gefüllt oder befüllbar ist. Dies erlaubt eine Anpassung an das Gewicht des Rührwerks.

Das Rührwerk ist vorzugsweise benachbart zur Revisionsöffnung mittels eines Anschlagbolzens an der Rührwerkshalterung fixierbar. Damit kann es für das Entnehmen fixiert werden.

Die Erfindung betrifft des Weiteren ein Verfahren zum Entnehmen eines Rührwerks aus einem durch eine Behälterwand seitlich umschlossenen Behälter, insbesondere einem Fermenter, insbesondere für eine Biogasanlage, wobei das Verfahren folgende Schritte aufweist:
- Verbringen des Rührwerks horizontal neben eine in der Behälterwand ausgebildete Revisionsöffnung,
- Öffnen der Revisionsöffnung,
- Befestigen des Rührwerks an einer Hebevorrichtung, welche an einem Galgen befestigt ist, wobei der Galgen an einer außenseitig oder innenseitig an der Behälterwand benachbart zur Revisionsöffnung angebrachten Galgenhalterung befestigt ist, einen zumindest teilweise über der Revisionsöffnung liegenden Querbalken aufweist und um eine vertikale Achse schwenkbar ist, und
- Entnehmen des Rührwerks aus dem Behälter mittels der Hebevorrichtung.

Mittels des erfindungsgemäßen Verfahrens kann ein Rührwerk auf einfache Weise seitlich entnommen werden, wobei auf das gemäß dem Stand der Technik meist nötige Abnehmen eines Behälterdachs und auf die Verwendung eines externen Krans verzichtet werden kann. Bezüglich weiterer Vorteile sei auf die obige Beschreibung des erfindugnsgemäßen Behälters verwiesen.

Insbesondere kann ein Behälter verwendet werden, welcher erfindungsgemäß ausgebildet ist. In diesem Fall kann bezüglich des Behälters auf alle beschriebenen Ausführungen und Varianten zurückgegriffen werden.

Das Rührwerk kann insbesondere mittels einer in dem Behälter befindlichen Rührwerkshalterung neben die Revisionsöffnung verbracht werden. Dies erlaubt ein leichtes Befesitgen an einer Vorrichtung zum Entnehmen des Rührwerks.

Das Rührwerk kann vor dem Schritt des Befestigens an einer Hebevorrichtung um eine vertikale Achse geschwenkt werden. Dies erlaubt das Verbringen des Rührwerks in eine Stellung, in welcher es leichter entnommen werden kann.

Das Verfahren weist bevorzugt einen Schritt des Befestigens eines Gegengewichts an einem Ende einer Horizontaltraverse der Hebevorrichtung auf. Damit kann die Horizontaltraverse vorteilhaft ins Gleichgewicht gebracht werden.

Vorteilhaft kann das Rührwerk durch Schwenken des Galgens um die vertikale Achse entnommen werden. Dies entspricht einer einfach zu realisiernden Bewegung.

Die Hebevorrichtung kann vorteilhaft als Horizontaltraverse ausgebildet sein. Dies emtmöglicht die bereits weiter oben beschriebene Vorgehensweise.

Die Hebevorrichtung ist gemäß einer bevorzugten Ausführung an dem Querbalken befestigt. Dadurch kann eine Bewegung der Hebevorrichtung vorteilhaft von der Bewegung des Galgens abgeleitet werden.

Das Rührwerk ist vorzugsweise im Behälter an einer Rührwerkshalterung entfernbar befestigt und wird von der Rührwerkshalterung vor dem Entnehmen entfernt. Die Rührwerkshalterung kann dabei insbesondere die bereits weiter oben beschriebene Funktionalität haben.

Die Erfindung betrifft des Weiteren eine Verwendung einer in einer seitlichen Behälterwand eines Behälters, insbesondere eines Fermenters, insbesondere für eine Biogasanlage, ausgebildeten Revisionsöffnung zur Entnahme eines in dem Behälter befindlichen Rührwerks, und zwar unter Verwendung eines Galgens, wobei der Galgen an einer außenseitig oder innenseitig an der Behälterwand benachbart zur Revisionsöffnung angebrachten Galgenhalterung befestigt ist, einen zumindest teilweise über der Revisionsöffnung liegenden Querbalken aufweist und um eine vertikale Achse schwenkbar ist.

Bezüglich der damit verbundenen Vorteile sei auf die obige Beschreibung verwiesen.

Der Behälter kann insbesondere erfindungsgemäß ausgebildet sein. Hinsichtlich eines erfindungsgemäßen Behälters kann auf alle beschriebenen Ausführungen und Varienten zurückgegriffen werden.

Vorteilhaft erfolgt die Entnahme in horizontaler Richtung. Dies hat sich als einfache Ausführung bewährt.

Die Entnahme kann insbesondere vorteilhaft mittels eines erfindungsgemäßen Verfahrens erfolgen. Dabei kann auf alle beschriebenen Ausführungen und Varianten zurückgegriffen werden.

In diesem Zusammenhang wird insbesondere darauf hingewiesen, dass alle im Bezug auf die Vorrichtung beschriebenen Merkmale und Eigenschaften aber auch Verfahrensweisen sinngemäß auch bezüglich der Formulierung des erfindungsgemäßen Verfahrens übertragbar und im Sinne der Erfindung einsetzbar und als mitoffenbart gelten. Gleiches gilt auch in umgekehrter Richtung, das bedeutet, nur im Bezug auf das Verfahren genannte, bauliche also vorrichtungsgemäße Merkmale können auch im Rahmen der Vorrichtungsansprüche berücksichtigt und beansprucht werden und zählen ebenfalls zur Offenbarung.

In der Zeichnung ist die Erfindung insbesondere in einem Ausführungsbeispiel schematisch dargestellt. Es zeigen:
- Fig. 1: eine perspektivische Ansicht eines Teils eines erfindungsgemäßen Behälters mit Rührwerk und Revisionsöffnung.
- Fig. 2: eine Außenansicht des erfindungsgemäßen Behälters.
- Fig. 3: eine weitere Außenansicht des erfindungsgemäßen Behälters.
- Fig. 4: eine Draufsicht auf den erfindungsgemäßen Behälter.

In den Figuren sind gleiche oder einander entsprechende Elemente jeweils mit den gleichen Bezugszeichen bezeichnet und werden daher, sofern nicht zweckmäßig, nicht erneut beschrieben. Die in der gesamten Beschreibung enthaltenen Offenbarungen sind sinngemäß auf gleiche Teile mit gleichen Bezugszeichen bzw. gleichen Bauteilbezeichnungen übertragbar. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiterhin können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

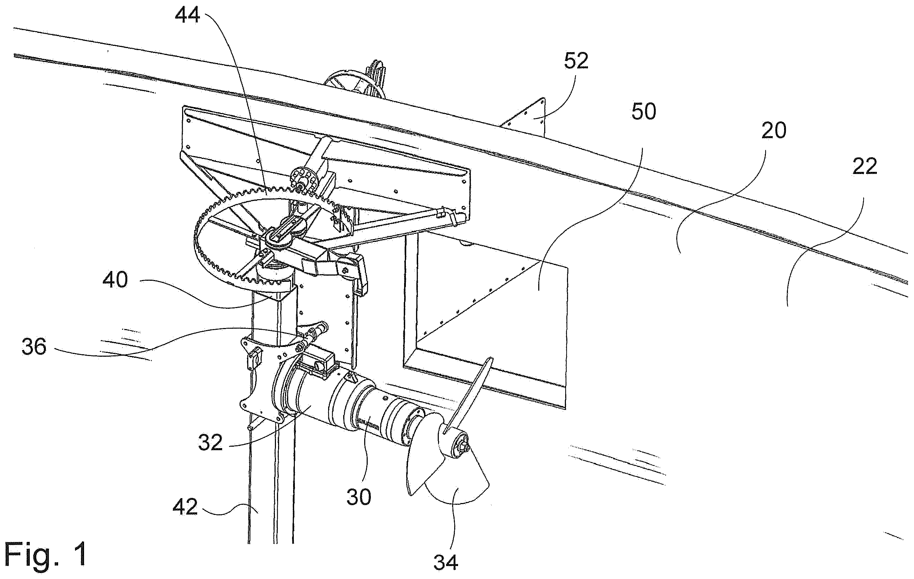

Figur 1 zeigt einen Teil eines Behälters 20, welcher gemäß einem Ausführungsbeispiel der Erfindung ausgestaltet ist. Der Behälter 20 ist vorliegend insbesondere ein Fermenter für eine Biogasanlage. Er ist überdacht, um erzeugtes Biogas zurückzuhalten und zur Verwendung abzuführen, was jedoch in den Figuren nicht dargestellt ist.

Der Behälter 20 weist eine Behälterwand 22 auf.

Der Behälter 20 weist im Inneren ein Rührwerk 30 auf. Das Rührwerk 30 ist aus einem Motor 32 und einem Propeller 34 gebildet. Es dient dazu, ein in dem Behälter 20 befindliches Biomassesubstrat zu durchmischen und damit für eine verbesserte Erzeugung von Biogas zu sorgen. Beispielsweise können verfestigte Bereiche aufgelöst werden.

Das Rührwerk 30 ist an einer Rührwerkshalterung 40 befestigt, welche wiederum an der Behälterwand 20 befestigt ist. Die Rührwerkshalterung 40 ist im Wesentlichen als vertikaler Mast 42 ausgebildet. Das Rührwerk ist an dem Mast 42 vertikal, auf einem Schlitten gelagert, verfahrbar, was es ermöglicht, auf unterschiedlichen Höhen im Behälter 20 die Rührwirkung des Rührwerks 30 zu verwenden.

Der Mast 42 ist wie gezeigt um seine vertikale Achse verdrehbar ausgebildet, was es ermöglicht, die Richtung einzustellen, in welcher die Rührwirkung erfolgen soll. Hierzu ist ein antreibbarer Zahnkranz 44 vorgesehen.

In der Behälterwand 22 ist eine seitliche Revisionsöffnung 50 ausgebildet. Diese dient dazu, das Rührwerk 30 für Wartungszwecke zu entnehmen. Hierzu kann insbesondere auf die nachfolgend beschriebene Vorgehensweise bzw. das entsprechende Verfahren zurückgegriffen werden. Die Revisionsöffnung 50 ist mittels einer Klappe 52 verschließbar, so dass während eines normalen Betriebs ein seitliches Entweichen von erzeugtem Biogas vermieden wird.

Das Rührwerk 30 kann entlang des Masts 42 in eine Position verbracht werden, in welcher sich das Rührwerk 30 unmittelbar horizontal benachbart zur Revisionsöffnung 50 befindet. Dies entspricht dem in Figur 1 dargestellten Zustand. Das Rührwerk ist in diesem Zustand mit einem Anschlagbolzen 36 fixierbar.

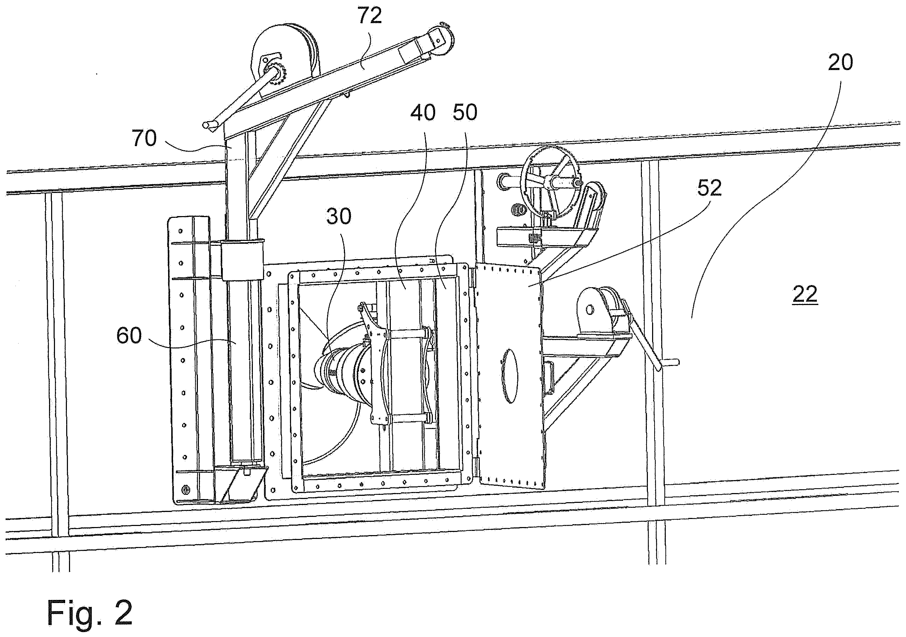

Figur 2 zeigt eine Außenansicht des Behälters 20. Dabei können durch die geöffnete Revisionsöffnung 50 das Rührwerk 30 und die Rührwerkshalterung 40 teilweise erkannt werden.

Außen an der Behälterwand 20 ist eine Galgenhalterung 60 angebracht. An dieser ist ein Galgen 70 befestigt, an dessen Oberseite ein Querbalken 72 ausgebildet ist. Der Galgen 70 ist um eine vertikale Achse schwenkbar, welche auch durch die Galgenhalterung 60 definiert ist. Dies kann in vorteilhafter Weise zum Entnehmen des Rührwerks 30 aus dem Behälter 20 verwendet werden.

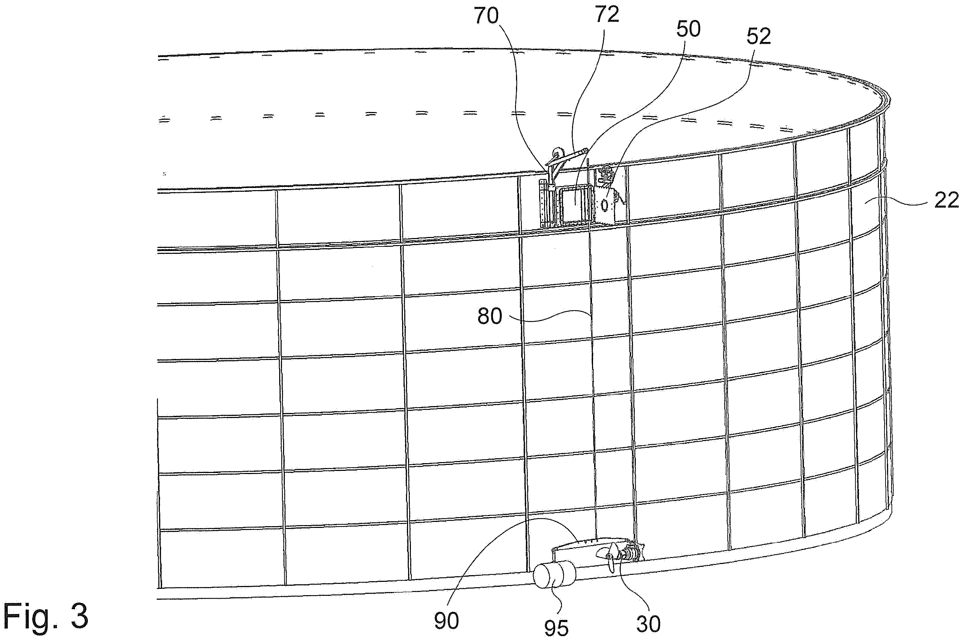

Figur 3 zeigt eine Außenansicht auf den Behälter 20 nach Entfernen des Rührwerks 30. Das Rührwerk 30 hängt dabei an einer Horizontaltraverse 90. Die Horizontaltraverse 90 ist wiederum an einem Seil 80 befestigt, welches seinerseits von dem Querbalken 72 des Galgens 70 herabhängt.

Die Horizontaltraverse 90 ist um eine horizontale Achse drehbar, welche durch den Aufhängepunkt an dem Seil 80 definiert ist. Wie gezeigt ist das Rührwerk 30 beabstandet zu diesem Aufhängepunkt angeordnet. Um die Horizontaltraverse 90 im Gleichgewicht zu halten, ist gegenüberliegend zum Rührwerk 30 ein Gegengewicht 95 befestigt. Dieses ist in Form eines Tanks ausgebildet, welcher mit Wasser als Ballaststoff befüllbar und vorliegend auch befüllt ist. Dabei wurde in das Gegengewicht 95 gerade so viel Wasser gefüllt, dass die Horizontaltraverse 90 im Gleichgewicht ist.

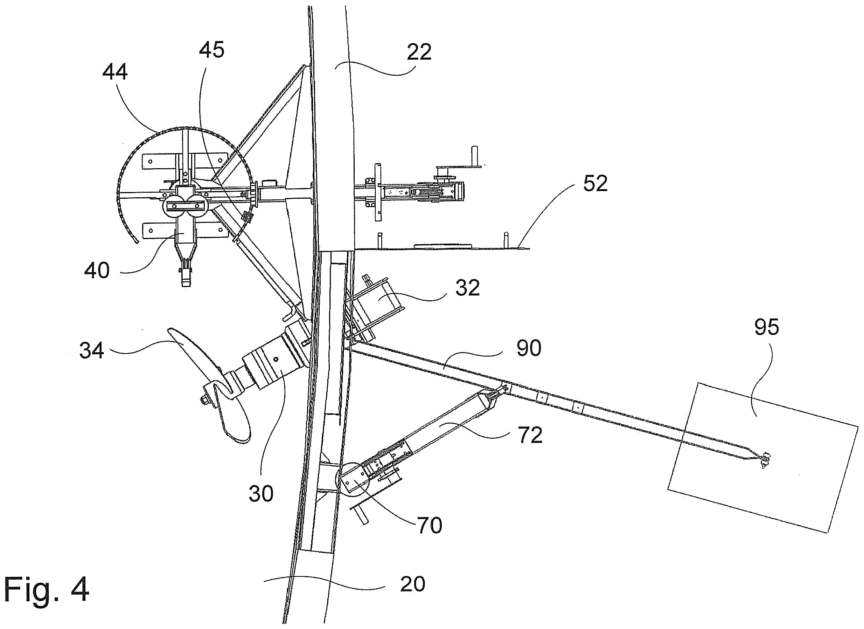

Figur 4 zeigt einen Zustand vor dem Entnehmen des Rührwerks 30 aus dem Behälter 20. Dabei ist das Rührwerk 30 an der Horizontaltraverse 90 befestigt. Außerdem ist das Gegengewicht 95 gegenüberliegend an der Horizontaltraverse 95 befestigt. Damit ist nun folgende Vorgehensweise möglich:
Das Gegengewicht 95 wird solange mit Wasser befüllt, bis sich die Horizontaltraverse 90 in einem drehmomentfreien Zustand befindet. Dies kann beispielsweise durch Kräftemessungen oder auch durch Beobachten festgestellt werden. Anschließend kann das Rührwerk von der Rührwerkshalterung 40 entfernt werden. Der Galgen 70 kann dann um seine vertikale Drehachse nach außen geschwenkt werden, so dass das Rührwerk 30 aus dem Behälter 20 entfernt wird. Die Klappe 52 kann anschließend geschlossen werden.

Die Horizontaltraverse 90 kann daraufhin beispielsweise abgesenkt werden, wodurch beispielsweise der in Figur 3 dargestellte Zustand erreicht werden kann.

Es sei verstanden, dass an dem Zahnkranz 44 insbesondere im Normalbetrieb, also bei normal verwendetem Rührwerk 30 ein klammerartiger Anschlag 45 angebracht sein kann, welcher ein Verstellen des Rührwerks 30 zu nahe an die Behälterwand 22 verhindert. Dabei kann vorteilhaft ein Entriegelungsbolzen vorgesehen sein, durch welchen die weitere Verstellung zum Entnehmen des Rührwerks dann ermöglicht werden kann. In Fig. 4 ist der Anschlag 45 in einer Position angeordnet, die nicht dem Normalbetrieb entspricht, sondern für das Entnehmen des Rührwerks dient.

Nachfolgend werden mögliche Merkmale des Vorschlages strukturiert wiedergegeben. Die nachfolgenden strukturiert wiedergegebenen Merkmale können beliebig untereinander kombiniert werden und können in beliebiger Kombination in die Ansprüche der Anmeldung aufgenommen werden. Dem Fachmann ist klar, dass sich die Erfindung bereits aus dem Gegenstand mit den wenigsten Merkmalen ergibt. Insbesondere sind nachfolgend vorteilhafte oder mögliche Ausgestaltungen, nicht jedoch die einzig möglichen Ausgestaltungen der Erfindung wiedergegeben.

Die Erfindung umfasst:
Einen Behälter, insbesondere Fermenter für eine Biogasanlage, der eine Behälterwand, welche den Behälter seitlich umschließt, aufweist und der Behälter ein Rührwerk aufweist, welches einen Motor und einen damit verbundenen Propeller aufweist, und der Fermenter eine Rührwerkshalterung aufweist, welche dazu ausgebildet ist, das Rührwerk zu tragen und an unterschiedliche Positionen innerhalb des Behälters zu verbringen, wobei in der Behälterwand eine verschließbare Revisionsöffnung ausgebildet ist, durch welche das Rührwerk entnehmbar ist, und die Rührwerkshalterung dazu ausgebildet ist, das Rührwerk in eine Position horizontal neben der Revisionsöffnung zu verbringen.

Den vorstehend genannten Behälter, wobei die Rührwerkshalterung dazu ausgebildet ist, dass das Rührwerk benachbart zur Revisionsöffnung von der Rührwerkshalterung entfernbar ist.

Den vorstehend genannten Behälter, wobei die Rührwerkshalterung als vertikaler Mast ausgebildet ist, entlang welchem das Rührwerk verfahrbar ist.

Den vorstehend genannten Behälter, wobei die Rührwerkshalterung zum Schwenken des Rührwerks um eine vertikale Achse ausgebildet ist.

Den vorstehend genannten Behälter, wobei außenseitig oder innenseitig an der Behälterwand, benachbart zur Revisionsöffnung, eine Galgenhalterung für einen Galgen angebracht ist.

Den vorstehend genannten Behälter, wobei in der Galgenhalterung ein Galgen befestigt ist, welcher einen zumindest teilweise über der Revisionsöffnung liegenden Querbalken aufweist.

Den vorstehend genannten Behälter, wobei der Galgen um eine vertikale Achse schwenkbar ist.

Den vorstehend genannten Behälter, wobei der Galgen eine Hebevorrichtung, insbesondere eine Horizontaltraverse trägt, welche zum Befestigen des im Behälter befindlichen Rührwerks durch die geöffnete Revisionsöffnung teilweise in den Behälter hineinreicht.

Den vorstehend genannten Behälter, wobei die Hebevorrichtung um eine horizontale Achse und/oder in einer horizontalen Ebene schwenkbar ist.

Den vorstehend genannten Behälter, wobei die Horizontaltraverse an einem ihrer Enden mit dem Rührwerk verbindbar ist und an einem gegenüberliegenden Ende mit einem Gegengewicht verbindbar ist, so dass die Horizontaltraverse zumindest im Wesentlichen ausbalanciert ist.

Den vorstehend genannten Behälter, wobei das Gegengewicht ein Behälter ist, welcher mit Ballaststoff, insbesondere mit Wasser, gefüllt oder befüllbar ist.

Den vorstehend genannten Behälter, wobei das Rührwerk benachbart zur Revisionsöffnung mittels eines Anschlagbolzens an der Rührwerkshalterung fixierbar ist.

Ein Verfahren zum Entnehmen eines Rührwerks aus einem durch eine Behälterwand seitlich umschlossenen Behälter, insbesondere eines Fermenters, insbesondere für eine Biogasanlage und insbesondere gemäß der Erfindung, wobei das Verfahren folgende Schritte aufweist:
- Verbringen des Rührwerks horizontal neben eine in der Behälterwand ausgebildete Revisionsöffnung,
- Öffnen der Revisionsöffnung,
- Befestigen des Rührwerks an einer Hebevorrichtung, und
- Entnehmen des Rührerks aus dem Behälter mittels der Hebevorrichtung.

Das vorstehend genannte Verfahren, wobei die Hebevorrichtung an einem Galgen befestigt ist, wobei der Galgen an einer außenseitig oder innenseitig an der Behälterwand benachbart zur Revisionsöffnung angebrachten Galgenhalterung befestigt ist, einen zumindest teilweise über der Revisionsöffnung liegenden Querbalken aufweist und um eine vertikale Achse schwenkbar ist.

Das vorstehend genannte Verfahren, wobei das Rührwerk mittels einer in dem Behälter befindlichen Rührwerkshalterung neben die Revisionsöffnung verbracht wird.

Das vorstehend genannte Verfahren, wobei das Rührwerk vor dem Schritt des Befestigens an einer Hebevorrichtung um eine vertikale Achse geschwenkt wird.

Das vorstehend genannte Verfahren, wobei das Verfahren einen Schritt des Befestigens eines Gegengewichts an einem Ende einer Horizontaltraverse der Hebevorrichtung aufweist.

Das vorstehend genannte Verfahren, wobei das Rührwerk durch Schwenken des Galgens um die vertikale Achse entnommen wird.

Das vorstehend genannte Verfahren, wobei die Hebevorrichtung als Horizontaltraverse ausgebildet ist.

Das vorstehend genannte Verfahren, wobei die Hebevorrichtung an dem Querbalken befestigt ist.

Das vorstehend genannte Verfahren, wobei das Rührwerk im Behälter an einer Rührwerkshalterung entfernbar befestigt ist und von der Rührwerkshalterung vor dem Entnehmen entfernt wird.

Eine Verwendung einer in einer seitlichen Behälterwand eines Behälters, insbesondere eines Fermenters, insbesondere für eine Biogasanlage und insbesondere gemäß der Erfindung, ausgebildeten Revisionsöffnung zur Entnahme eines in dem Behälter befindlichen Rührwerks.

Die vorstehend genannte Verwendung, welche unter Verwendung eines Galgens erfolgt, wobei der Galgen an einer außenseitig oder innenseitig an der Behälterwand benachbart zur Revisionsöffnung angebrachten Galgenhalterung befestigt ist, einen zumindest teilweise über der Revisionsöffnung liegenden Querbalken aufweist und um eine vertikale Achse schwenkbar ist.

Die vorstehend genannte Verwendung, wobei die Entnahme in horizontaler Richtung erfolgt.

Die vorstehend genannte Verwendung, wobei die Entnahme mittels eines erfindungsgemäßen Verfahrens erfolgt.

Die jetzt mit der Anmeldung und später eingereichten Ansprüche sind ohne Präjudiz für die Erzielung weitergehenden Schutzes.

Sollte sich hier bei näherer Prüfung, insbesondere auch des einschlägigen Standes der Technik, ergeben, dass das eine oder andere Merkmal für das Ziel der Erfindung zwar günstig, nicht aber entscheidend wichtig ist, so wird selbstverständlich schon jetzt eine Formulierung angestrebt, die ein solches Merkmal, insbesondere im Hauptanspruch, nicht mehr aufweist. Auch eine solche Unterkombination ist von der Offenbarung dieser Anmeldung abgedeckt.

Es ist weiter zu beachten, dass die in den verschiedenen Ausführungsformen beschriebenen und in den Figuren gezeigten Ausgestaltungen und Varianten der Erfindung beliebig untereinander kombinierbar sind. Dabei sind einzelne oder mehrere Merkmale beliebig gegeneinander austauschbar. Diese Merkmalskombinationen sind ebenso mit offenbart.

Die in den abhängigen Ansprüchen angeführten Rückbeziehungen weisen auf die weitere Ausbildung des Gegenstandes des Hauptanspruches durch die Merkmale des jeweiligen Unteranspruches hin. Jedoch sind diese nicht als ein Verzicht auf die Erzielung eines selbständigen, gegenständlichen Schutzes für die Merkmale der rückbezogenen Unteransprüche zu verstehen.

Merkmale, die nur in der Beschreibung offenbart wurden oder auch Einzelmerkmale aus Ansprüchen, die eine Mehrzahl von Merkmalen umfassen, können jederzeit als von erfindungswesentlicher Bedeutung zur Abgrenzung vom Stande der Technik in den oder die unabhängigen Anspruch/Ansprüche übernommen werden, und zwar auch dann, wenn solche Merkmale im Zusammenhang mit anderen Merkmalen erwähnt wurden beziehungsweise im Zusammenhang mit anderen Merkmalen besonders günstige Ergebnisse erreichen.

## Patentansprüche

1. Behälter, insbesondere Fermenter für eine Biogasanlage, der eine Behälterwand (22), welche den Behälter (20) seitlich umschließt, aufweist und der Behälter ein Rührwerk (30) aufweist, welches einen Motor (32) und einen damit verbundenen Propeller (34) aufweist, und der Behälter eine Rührwerkshalterung (40) aufweist, welche dazu ausgebildet ist, das Rührwerk (30) zu tragen und an unterschiedliche Positionen innerhalb des Behälters (20) zu verbringen, wobei in der Behälterwand (22) eine verschließbare Revisionsöffnung (50) ausgebildet ist, durch welche das Rührwerk (30) entnehmbar ist, und die Rührwerkshalterung (40) dazu ausgebildet ist, das Rührwerk (30) in eine Position horizontal neben der Revisionsöffnung (50) zu verbringen, **dadurch gekennzeichnet, dass** außenseitig oder innenseitig an der Behälterwand (22), benachbart zur Revisionsöffnung (50) eine Galgenhalterung (60) für einen Galgen angebracht ist, wobei in der Galgenhalterung (60) ein Galgen (70) befestigt ist, welcher einen zumindest teilweise über der Revisionsöffnung (50) liegenden Querbalken (72) aufweist, und wobei der Galgen (70) um eine vertikale Achse schwenkbar ist.

2. Behälter nach Anspruch 1, **dadurch gekennzeichnet, dass** die Rührwerkshalterung (40) dazu ausgebildet ist, dass das Rührwerk (30) benachbart zur Revisionsöffnung (50) von der Rührwerkshalterung (40) entfernbar ist.

3. Behälter nach einem der vorhergehenden Ansprüche, **dadurch gekennzeichnet, dass** die Rührwerkshalterung (40) als vertikaler oder schräger Mast (42) ausgebildet ist, entlang welchem das Rührwerk (30) verfahrbar ist.

4. Behälter nach einem der vorhergehenden Ansprüche, **dadurch gekennzeichnet, dass** der Galgen (70) eine Hebevorrichtung, insbesondere eine Horizontaltraverse (90), trägt, welche zum Befestigen des im Behälter (20) befindlichen Rührwerks (30) durch die geöffnete Revisionsöffnung (50) teilweise in den Behälter (20) hineinreicht.

5. Behälter nach Anspruch 4, **dadurch gekennzeichnet, dass** die Horizontaltraverse (90) an einem ihrer Enden mit dem Rührwerk (30) verbindbar ist und an einem gegenüberliegenden Ende mit einem Gegengewicht (95) verbindbar ist, so dass die Horizontaltraverse (90) zumindest im Wesentlichen ausbalanciert ist.

6. Behälter nach Anspruch 5, **dadurch gekennzeichnet, dass** das Gegengewicht (95) ein Behälter ist, welcher mit Ballaststoff, insbesondere mit Wasser, gefüllt oder befüllbar ist.

7. Verfahren zum Entnehmen eines Rührwerks aus einem durch eine Behälterwand seitlich umschlossenen Behälter, insbesondere einem Fermenter, insbesondere für eine Biogasanlage, wobei das Verfahren folgende Schritte aufweist:
Verbringen des Rührwerks horizontal neben eine in der Behälterwand ausgebildete Revisionsöffnung,
Öffnen der Revisionsöffnung,
Befestigen des Rührwerks an einer Hebevorrichtung, welche an einem Galgen befestigt ist, wobei der Galgen an einer außenseitig oder innenseitig an der Behälterwand benachbart zur Revisionsöffnung angebrachten Galgenhalterung befestigt ist, einen zumindest teilweise über der Revisionsöffnung liegenden Querbalken aufweist und um eine vertikale Achse schwenkbar ist, und
Entnehmen des Rührwerks aus dem Behälter mittels der Hebevorrichtung.

8. Verfahren nach Anspruch 7, **dadurch gekennzeichnet, dass** der Behälter nach einem der Ansprüche 1 bis 6 ausgebildet ist.

9. Verfahren nach einem der Ansprüche 7 oder 8, **dadurch gekennzeichnet, dass** das Rührwerk durch Schwenken des Galgens um die vertikale Achse entnommen wird.

10. Verfahren nach einem der Ansprüche 7 bis 9, **dadurch gekennzeichnet, dass** die Hebevorrichtung als Horizontaltraverse ausgebildet ist.

11. Verfahren nach einem der Ansprüche 7 bis 10, **dadurch gekennzeichnet, dass** die Hebevorrichtung an dem Querbalken befestigt ist.

12. Verfahren nach einem der Ansprüche 7 bis 11, **dadurch gekennzeichnet, dass** das Rührwerk im Behälter an einer Rührwerkshalterung entfernbar befestigt ist und von der Rührwerkshalterung vor dem Entnehmen entfernt wird.

13. Verwendung einer in einer seitlichen Behälterwand eines Behälters, insbesondere eines Fermenters, insbesondere für eine Biogasanlage, ausgebildeten Revisionsöffnung zur Entnahme eines in dem Behälter befindlichen Rührwerks, und zwar unter Verwendung eines Galgens, wobei der Galgen an einer außenseitig oder innenseitig an der Behälterwand benachbart zur Revisionsöffnung angebrachten Galgenhalterung befestigt ist, einen zumindest teilweise über der Revisionsöffnung liegenden Querbalken aufweist und um eine vertikale Achse schwenkbar ist.

14. Verwendung nach Anspruch 13, **dadurch gekennzeichnet, dass** der Behälter nach einem der Ansprüche 1 bis 6 ausgebildet ist.

15. Verwendung nach einem der Ansprüche 13 oder 14, **dadurch gekennzeichnet, dass** die Entnahme in horizontaler Richtung erfolgt.

## Claims

1. A container, in particular fermenter for a biogas plant, with a container wall (22) laterally encircling the container (20), and with a mixer (30) provided with a motor (32) and a propeller (34) connected thereto, and the container being provided with a mixer mount (40) configured for carrying the mixer (30) and moving it in different positions within the container (20), wherein in the container wall (22) a sealable inspection opening (50) is formed, through which the mixer (30) can be removed, and the mixer mount (40) is configured for moving the mixer (30) in a position horizontally next to the inspection opening (50), **characterized in that** either outside or inside to the container wall (22) adjacent to the inspection opening (50) a boom mounting (60) for a boom is attached, wherein in the boom mounting (60) a boom (70) is installed that has a crossbeam (72) extending at least partly over the inspection opening (72), and wherein the boom (70) can be swung around a vertical axis.

2. The container according to claim 1, **characterized in that** the mixer mount (40) is formed such that the mixer (30) adjacent to the inspection opening (50) is removable from the mixer mount (40).

3. The container according to any one of the preceding claims, **characterized in that** the mixer mount (40) is formed as vertical or transverse mast (42) and the mixer (30) travels along it.

4. The container according to any one of the preceding claims, **characterized in that** the boom (70) carries a lifting device in particular a horizontal traverse crossbeam (90) extending partly into the container (20) through the open inspection opening (50) for attaching the mixer (30) located in the container (20).

5. The container according to claim 4, **characterized in that** the horizontal traverse crossbeam (90) is connectable on one of its ends with the mixer (30), and on one opposite end with a counter weight (95), so that the horizontal traverse crossbeam (90) is essentially balanced.

6. The container according to claim 5, **characterized in that** the counter weight (95) is a container filled or fillable with ballast, in particular water.

7. A method for removing a mixer from a container laterally encircled by a container wall, in particular a fermenter, in particular for a biogas plant, wherein the method comprises the following steps:
- Moving the mixer horizontally next to an inspection opening in the container wall,
- Opening the inspection opening,
- Attaching the mixer to a lifting device that is attached to a boom, wherein the boom is installed on a boom mounting attached either outside or inside the container wall adjacent to the inspection opening, has a crossbeam extending at least partly over the inspection opening, and can be swung around a vertical axis, and
- Removing the mixer from the container by means of the lifting device.

8. The method according to claim 7, **characterized in that** the container is designed according to any one of the claims 1 to 6.

9. The method according to either claim 7 or 8, **characterized in that** the mixer is removed by swinging the boom around the vertical axis.

10. The method according to any one of the claims 7 to 9, **characterized in that** the lifting device is formed as horizontal traverse crossbeam.

11. The method according to any one of the claims 7 to 10, **characterized in that** the lifting device is attached to the crossbeam.

12. The method according to any one of the claims 7 to 11, **characterized in that** the mixer is attached removably to a mixer mount in the container, and is taken off the mixer mount before removal.

13. A use of an inspection opening formed in a lateral container wall of a container, in particular a fermenter, in particular for a biogas plant, for the removal of a mixer located in the container, and this is by using a boom, wherein the boom is installed in a boom mounting provided either outside or inside at the container wall adjacent to the inspection opening, has a crossbeam extending at least partly over the inspection opening, and can swing around a vertical axis.

14. The use according to claim 13, **characterized in that** the container is designed according to any one of the claims 1 to 6.

15. The use according to either claim 13 or 14, **characterized in that** removal is carried out in horizontal direction.

## Revendications

1. Récipient, en particulier un fermenteur, destiné à une centrale de biogaz, comportant une paroi de récipient (22) enfermant latéralement le récipient (20), un mélangeur (30) muni d'un moteur (32) et d'une hélice (34) liée à ce moteur, un support de mélangeur (40) réalisé de façon à pouvoir maintenir le mélangeur (30) et de le déplacer au niveau de différentes positions à l'intérieur du récipient (20), et une trappe de révision (50) située dans la paroi de récipient (22) et pouvant être fermée et permettant de retirer le mélangeur (30), de façon à ce que la configuration du support de mélangeur (40) permet de déplacer le mélangeur (30) en une position horizontale à côté de la trappe de révision (50), **caractérisé en ce qu'**une fixation de potence (60) pour une potence est située à l'intérieur ou à l'extérieur sur la paroi de récipient (22) à côté de la trappe de révision (50) de façon à ce que la potence (70) fixée par la fixation de potence (60) comporte une traverse (72) située au moins partiellement au-dessus de la trappe de révision (50) et à ce que la potence (70) peut pivoter autour d'un axe vertical.

2. Récipient selon la revendication 1, **caractérisé en ce que** le support de fixation (40) est configuré de façon à ce que le mélangeur peut être enlevé de la fixation de mélangeur (40) à proximité de la trappe de révision (50).

3. Récipient selon une des revendication précédentes, **caractérisé en ce que** le support de mélangeur (40) est configuré comme un mât vertical ou incliné le long duquel le mélangeur (30) peut coulisser.

4. Récipient selon une des revendications précédentes, **caractérisé en ce que** la potence (70) supporte un dispositif de levage, en particulier une traverse horizontale (90), partiellement traversant la trappe de révision (50) vers l'intérieur du récipient afin de pouvoir fixer le mélangeur (30) situé à l'intérieur du récipient (20).

5. Récipient selon la revendication 4, **caractérisé en ce que** la traverse horizontale (90) peut être fixée par une de ses extrémités au mélangeur (30) et par l'extrémité opposée à un contre-poids (95) afin d'obtenir essentiellement un équilibre de la traverse horizontale (90).

6. Récipient selon la revendication 5, **caractérisé en ce que** le contre-poids (95) est un récipient rempli ou pouvant être rempli d'un ballaste, en particulier d'eau.

7. Procédé d'enlèvement d'un mélangeur d'un récipient latéralement fermé par une paroi de récipient, en particulier un fermenteur, en particulier destiné à une centrale de biogaz, le procédé comportant les étapes suivantes :
- déplacement horizontalement du mélangeur au voisinage d'une trappe de révision située dans la paroi de récipient,
- ouverture de la trappe de révision,
- fixation du mélangeur à un dispositif de levage fixé à une potence qui est maintenue par une fixation de potence située à l'intérieur ou à l'extérieur de la paroi de récipient et à proximité de la trappe de révision et comportant une traverse située au moins partiellement au-dessus de la trappe de révision et pouvant pivoter autour d'un axe vertical,
- et l'enlèvement du mélangeur du récipient à l'aide du dispositif de levage.

8. Procédé selon la revendication 7, **caractérisé en ce que** le récipient est configuré selon une des revendications 1 à 6.

9. Procédé selon une des revendications 7 ou 8, **caractérisé en ce que** le mélangeur est enlevé par pivotement de la potence autour de l'axe vertical.

10. Procédé selon une des revendications 7 à 9, caractérisé à ce que le dispositif de levage est configuré comme une traverse horizontale.

11. Procédé selon une des revendications 7 à 10, **caractérisé en ce que** le dispositif de levage est fixé à la traverse.

12. Procédé selon une des revendications 7 à 11, **caractérisé en ce que** le mélangeur situé à l'intérieur du récipient est maintenu de façon amovible par une fixation de mélangeur et peut ainsi être désolidarisé de sa fixation avant son enlèvement.

13. Utilisation d'une trappe de révision située dans une paroi de récipient latérale, en particulier d'un fermenteur, en particulier d'une centrale à biogaz, destinée à permettre l'enlèvement d'un mélangeur situé à l'intérieur du récipient en utilisant une potence, qui est maintenue par une fixation de potence fixée à l'intérieur ou à l'extérieur de la paroi de récipient à proximité de la trappe de révision, possédant une traverse située au moins partiellement au-dessus de la trappe de révision et pouvant pivoter autour d'un axe vertical.

14. Utilisation selon la revendication 13, **caractérisé en ce que** le récipient est configuré selon une des revendications 1 à 6.

15. Utilisation selon une des revendications 13 ou 14, **caractérisé en ce que** l'enlèvement s'effectue selon une direction horizontale.
